# EUROPEAN PATENT SPECIFICATION

(11) **EP 2 968 799 B1**
(45) Date of publication and mention of the grant of the patent: **31.05.2017**
(21) Application number: 14779418.4
(22) Date of filing: 12.03.2014
(51) Int. Cl.: A61M 11/00, A61M 16/14, A61M 16/10, A61M 15/00, A61M 11/06, A61M 16/00

(54) **SINGLE-PIECE AEROSOL EXHALATION FILTER AND RESPECTIVE AEROSOL DELIVERY DEVICE**
EINTEILIGER AEROSOLAUSATMUNGSFILTER UND DAZUGEHÖRIGE AEROSOLABGABEVORRICHTUNG
FILTRE D'EXHALATION D'AÉROSOL MONOBLOC ET DISPOSITIF D'ADMINISTRATION D'AÉROSOL CORRESPONDANT

(30) Priority: 13.03.2013 US 201361779521 P
(43) Date of publication of application: 20.01.2016
(73) Proprietor: Teleflex Medical Incorporated, Morrisville, North Carolina 27560 (US)
(72) Inventor: BURK, Marc, Menifee, California 92584 (US); DWYER, Daniel Patrick, Raleigh, North Carolina 27613 (US)
(74) Representative: Cordina, Kevin John
(86) International application number: PCT/US2014/024073
(87) International publication number: WO 2014/164997

(56) References cited:
- WO-A1-98/07464
- US-A- 4 064 876
- US-A- 5 020 530
- US-A- 5 044 361
- US-A- 5 044 361
- US-A- 5 603 314
- US-A- 5 603 314
- US-A- 5 686 050
- US-A1- 2003 106 555
- US-A1- 2005 217 666
- US-A1- 2007 101 994
- US-A1- 2008 310 994
- US-A1- 2010 095 958
- US-A1- 2011 108 025

## Description

### TECHNICAL FIELD

This disclosure generally relates to preventing medicaments produced by an aerosol delivery device from entering the atmosphere. More particularly, this disclosure relates to a single-piece aerosol exhalation filter connected to an exhaust port of an aerosol delivery device for filtering droplets of a liquid medicament from the exhalation of a patient.

### BACKGROUND

Aerosols including medicaments, such as anesthetics, are commonly administered to patients. The aerosols may be administered to the patients using an aerosol delivery device, such as a nebulizer or atomizer. The aerosol delivery device can atomize a liquid medicament and the resulting droplets of the liquid medicament will mix with atmospheric air and/or a particular gas from a pressurized gas source for inhalation by the patient. During subsequent exhalation, the patient will breathe out at least some of the droplets of the liquid medicament that were not absorbed by the patient.

Small droplets of medicaments can hang suspended in the atmosphere of, for example, hospital rooms for several hours, putting both medical practitioners and other patients at risk. For example, medical practitioners continuously exposed to even low doses of an anesthetic can lose consciousness or have diminished capacity to provide competent care to the patients under their care. In addition, studies have shown that continuous exposure to bronchodilators has led to occupational asthma in medical practitioners. To prevent medicaments from entering the atmosphere, various exhalation filters have been developed to capture droplets of the medicaments from the exhalation of patients using aerosol delivery devices.

Existing exhalation filters, however, are typically constructed of multiple pieces. For example, many existing exhalation filters include a plastic body that removably receives a porous sheet. Such multi-piece exhalation filters are relatively costly to manufacture, are relatively heavy, require knowledge of assembly of the exhalation filters, and require knowledge of connection of the exhalation filters to their respective aerosol delivery devices. Given the rise in the use of disposable, handheld, small volume aerosol delivery devices, such additional cost, weight, and specialized knowledge of assembly and connection may prevent the use of exhalation filters, resulting in risks associated with the second-hand exposure of the medicaments.

Therefore, a need exists for a single-piece aerosol exhalation filter that is less costly to manufacture, lighter, and easier to use in aerosol delivery devices, such as disposable, handheld, small volume aerosol delivery devices.

US2008/310994 discloses a breathing assistance apparatus with a patient interface, and including humidification and disinfection devices.

### SUMMARY

The foregoing needs are met, to a great extent, by implementations of the single-piece aerosol exhalation filter and the aerosol delivery device including the single-piece aerosol exhalation filter for filtering droplets of a liquid medicament from the exhalation of a patient.

There is provided an aerosol delivery device for delivering an aerosol to a patient, the aerosol delivery device comprising: a nebulizer configured to generate an aerosol containing droplets of a liquid medicament; an inhalation conduit defining an inhalation passage, the inhalation passage configured to allow passage of the aerosol generated by the nebulizer to the patient; an exhalation conduit defining an exhalation passage, the exhalation passage configured to allow passage of a gaseous or fluid medium exhaled from the patient, the gaseous or fluid medium comprising droplets of the liquid medicament; and a single-piece filter having an internal volume between 5 mL and 200 mL and an axial length between 7.5 cm and 30 cm connected to the exhalation conduit, the single-piece filter configured to capture at least some of the droplets of the liquid medicament from the gaseous or fluid medium, wherein: the single-piece filter comprises a continuous, single-piece filter body having a proximal portion and a distal portion, the proximal portion of the filter body has a proximal end that defines an opening having a diameter between 15 mm and 30 mm configured to connect to the exhalation conduit, and the distal portion of the filter body has a closed distal end.

A selection of optional features is set out in the dependent claims.

In this respect, before explaining at least one implementation in detail, it is to be understood that the single-piece aerosol exhalation filter and the aerosol delivery device including the single-piece aerosol exhalation filter are not limited in their application to the details of construction and to the arrangements of the components set forth in the following description or illustrated in the drawings. Also, it is to be understood that the phraseology and terminology employed herein, as well as the Abstract, are for the purpose of description and should not be regarded as limiting.

As such, those skilled in the art will appreciate that the conception upon which this disclosure is based may readily be utilized as a basis for the designing of other structures, methods, and systems for carrying out the several purposes of the single-piece aerosol exhalation filter and the aerosol delivery device including the single-piece aerosol exhalation filter. It is understood, therefore, that the claims include such equivalent constructions insofar as they do not depart from the scope of the present application.

### BRIEF DESCRIPTION OF THE DRAWINGS

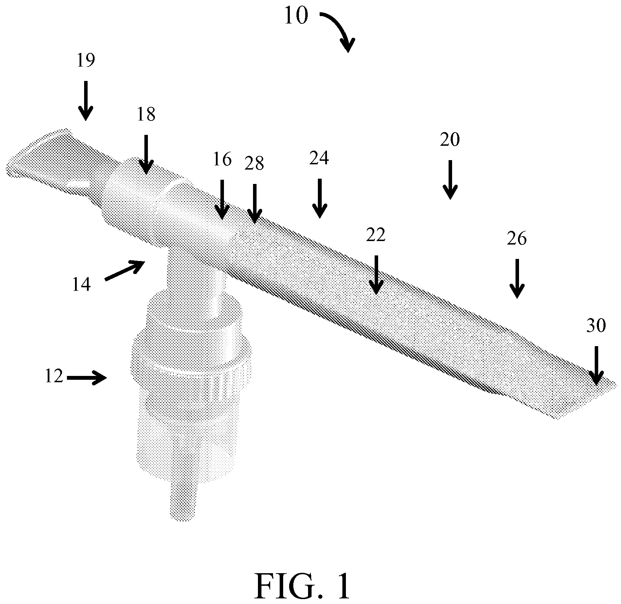

FIG. 1 is a perspective view illustrating an exemplary aerosol delivery device including a single-piece aerosol exhalation filter.

### DETAILED DESCRIPTION

Implementations of the single-piece aerosol exhalation filter and the aerosol delivery device including the single-piece aerosol exhalation filter are described with reference to the drawings, in which like reference numerals refer to like parts throughout.

Referring to FIG. 1, an aerosol delivery device **10** including a single-piece aerosol exhalation filter **20** is illustrated. The aerosol delivery device **10** includes a nebulizer **12,** a connecter **14** having a tee-shape defining an exhalation conduit **16** and an inhalation conduit **18** at opposite ends of the connecter **14,** a mouthpiece **19** connected to the inhalation conduit **18,** and the single-piece aerosol exhalation filter **20** connected to the exhalation conduit **16.** An exhaust port can be located at the distal end of the exhalation conduit **16** and is covered by the single-piece aerosol exhalation filter **20.** The exhalation conduit **16** defines an exhalation passage or lumen while the inhalation conduit **18** defines an inhalation passage or lumen. The inhalation passage can be in fluid communication with the nebulizer **12** and the exhalation passage can be in fluid communication with the inhalation passage.

The single-piece aerosol exhalation filter **20** includes a continuous, single-piece body **22** having a proximal portion **24** and a distal portion **26.** The single-piece aerosol exhalation filter **20** is made completely of a macroporous or microporous material that captures the droplets of the liquid medicament from the patient's breath, thereby filtering the patient's breath to prevent the droplets from entering the atmosphere. The proximal portion 24 has a proximal end **28** that defines an opening configured to connect to the exhalation conduit **16.** The distal portion **26** has a closed distal end **30** that does not allow any gaseous or fluid medium to escape without passing through the single-piece aerosol exhalation filter **20.**

The nebulizer **12** produces an aerosol by generating droplets of a liquid medicament that are mixed with pressurized air and/or atmospheric air. The pressurized air can be, for example, oxygen or the like used for atomization of the liquid medicament. The aerosol travels through the connecter **14** defining the inhalation conduit **18** and the mouthpiece **19** to deliver the medicament to the patient during inhalation. As the patient exhales, the patient's breath, including droplets of the liquid medicament that were not absorbed by the patient, travels through the mouthpiece **19,** the connecter **14** defining the inhalation conduit **18** and the exhalation conduit **16,** and to the single-piece aerosol exhalation filter **20.** The droplets of the liquid medicament will then be captured by the single-piece aerosol exhalation filter **20** while the gases of the patient's breath will pass through the single-piece aerosol exhalation filter **20** to the atmosphere.

To allow the patient to breathe without restriction, the material used to form the single-piece body **22** may allow low resistance gas flow in both directions. In particular, the single-piece aerosol exhalation filter **20** may have sufficient dimensions and resistance to enable a patient with weak or compromised breathing to breathe through the aerosol delivery device **10** for up to 60 minutes without fatigue. In some implementations, the flow resistance through the single-piece aerosol exhalation filter **20** may be less than, for example, five cm·H₂O when 60 LPM is directed through the single-piece aerosol exhalation filter **20.**

In addition to allowing low resistance gas flow, however, the single-piece aerosol exhalation filter **20** can be made of a material with sufficient density to trap a majority of the droplets of the liquid medicament in the patient's breath. In some implementations, the material used to form the single-piece body **22** may trap over 90% and, preferably, over 99% of the droplets of the liquid medicament.

If the material is used to form the single-piece body **22** is microporous, the material can have pores with diameters between two nanometers and 50 nanometers. The pore size of the material used to form the single-piece body **22** should be small enough to capture the droplets of the liquid medicament within the material or to prevent the droplets from passing through the material.

In some implementations, the material used to form the single-piece body **22** can have, for example, an N95 rating capable of filtering at least 95% of airborne particles with aerodynamic diameters of 0.3 micrometres or greater, an N99 rating capable of filtering at least 99% of airborne particles, or an N100 rating capable of filtering at least 99.7% of airborne particles, i.e., High-Efficiency Particulate Air ("HEPA") rating. Preferably, the material used to form the single-piece body **22** can be capable of filtering at least 95% of the airborne particles. The material may or may not be oil resistant.

The material may include strands of woven or non-woven material which can be layered to acquire a desired filtration density of, for example, between 30 and 300 g/cm³. The strands can be thermoplastic strands made of polypropylene, polyethylene, or the like. The material can have sufficient mechanical strength to support the weight of the single-piece aerosol exhalation filter **20** without additional support. In some implementations, one layer of the material can be used to form the single-piece body **22,** whereas in other implementations, multiple layers of the material can be wrapped to form the single-piece body **22.**

As illustrated in FIG. 1, the proximal portion **24** of the single-piece body **22** can have a generally tubular, i.e., cylindrical, shape with a circular cross section. In some implementations, however, the proximal portion **24** of the single-piece body **22** can have an elliptical cross section, a square cross section, a rectangular cross section, a pentagon cross section, or the like. The cross section of the proximal portion **24** of the single-piece body **22** will depend on the cross section of the exhalation conduit **16,** such that the cross section of the proximal portion **24** of the single-piece body **22** and the cross section of the exhalation conduit **16** can preferably be the same. By having the same cross section shape, the patient's breath will pass from the connecter **14** to the single-piece aerosol exhalation filter **20** without escaping and a friction fit of sufficient strength is assured.

The single-piece body **22** may have a shape that can support itself, such that the single-piece aerosol exhalation filter **20** does not require additional support, such as a frame. By not requiring an additional support, the single-piece aerosol exhalation filter can be manufactured more economically as a single piece. In addition to being self-supporting, a tubular shape can also be manufactured more economically relative to other shapes. Moreover, because many aerosol delivery devices have an exhalation conduit with a circular cross section, the tubular shape of the single-piece aerosol exhalation filter **20** enables it to be used in combination with a variety of aerosol delivery devices.

The nebulizer **12,** connecter **14,** and mouthpiece **19** can be made of one or more substantially rigid materials such as, plastic including, for example, acrylic, polyethylene, polyamide, or polyvinyl chloride; metals including, for example, steel or aluminum; and/or combinations thereof.

The single-piece aerosol exhalation filter **20** can be connected to the exhaust port of the exhalation conduit **16** using one or more of a variety of different connections means, such as, for example, friction fit, adhesives, tongue and grooves, rubber bands, zip ties, nails, screws, clips, or the like. In preferred implementations, the single-piece aerosol exhalation filter **20** is connected to the exhaust port by friction fit. For example, where the outer diameter of the single-piece aerosol exhalation filter **20** is equal to the outer diameter of the exhalation conduit **16,** the outer diameter of the exhaust port can be smaller than the outer diameter of the exhalation conduit **16.** In particular, the outer diameter of the exhaust port can be equal to the outer diameter of the exhalation conduit **16** minus twice the thickness of the body of the single-piece aerosol exhalation filter **20.** In another example, the inner diameter of the single-piece aerosol exhalation filter **20** can be equal to the outer diameter of the exhaust port to enable a friction fit between the two.

In some implementations, the exhaust port at the distal end of the exhalation conduit **16** can have a circular cross section with an outer diameter between 15 and 30 mm and, preferably, about 22 mm. As such, the opening at the proximal end **28** of the single-piece aerosol exhalation filter **20** can also have a circular cross section with an inner diameter of about 22 mm to enable a friction fit connection of the single-piece aerosol exhalation filter **20** to the exhalation conduit **16.**

The internal volume of the single-piece aerosol exhalation filter **20** should be sufficient to collect the droplets of the liquid medicament that would otherwise be wasted and harmful to others. In some implementations, the internal volume of the single-piece aerosol exhalation filter **20** can be between five mL and 200 mL. Testing has shown that an internal volume of about 50 mL is sufficient to collect the droplets of the liquid medicament from a patient's breath.

The axial length of the single-piece aerosol exhalation filter **20** can depend on the desired internal volume of the single-piece aerosol exhalation filter **20** and the diameter of the exhaust port of the aerosol delivery device **10.** In some implementations, the axial length of the single-piece aerosol exhalation filter **20** can be between 7.5 cm and 30 cm. Preferably, however, to manufacture a single-piece aerosol exhalation filter **20** with an internal volume of about 50 mL having an opening defined by the proximal end **28** with a diameter of 22 mm, the axial length may be about 15 cm.

In some implementations, the closed distal end **30** of the single-piece aerosol exhalation filter **20** can be formed by bonding the opposite sides of the distal portion **26** together after the tubular single-piece body **22** is formed. The opposite sides of the distal portion **26** can be bonded together using an adhesive, heat fusion, chemical fusion, or the like. In other implementations, the closed distal end **30** can be originally formed as a closed end during manufacturing. For example, the single-piece aerosol exhalation filter **20** can be extruded with a closed distal end **30.**

The many features and advantages of the single-piece aerosol exhalation filter 20 are apparent from the detailed specification, and thus, the claims cover all such features and advantages within the scope of this application. Further, numerous modifications and variations are possible.

For example, in addition to filtering the droplets of the liquid medicament, the single-piece aerosol exhalation filter **20** can also be configured to filter bacteria molecules and viruses present in the patient's breath. In particular, the material forming the single-piece body **22** can be selected to have suitable pore sizes effective for filtering the droplets of the liquid medicament, as well as bacteria and viruses.

In another example, the aerosol delivery device **10** can include one or more one-way valves in the exhalation conduit **16** and/or the inhalation conduit **18** to block the fluid communication of one or more passages. For example, the fluid communication between the nebulizer **12** and the inhalation passage can be blocked during exhalation using a one-way valve in the connecter **14.**

As such, it is not desired to limit the single-piece aerosol exhalation filter **20** to the exact construction and operation described and illustrated, and accordingly, all suitable modifications and equivalents may fall within the scope of the claims.

## Claims

1. An aerosol delivery device for delivering an aerosol to a patient, the aerosol delivery device comprising:
a nebulizer (12) configured to generate an aerosol containing droplets of a liquid medicament;
an inhalation conduit (18) defining an inhalation passage, the inhalation passage configured to allow passage of the aerosol generated by the nebulizer (12) to the patient;
an exhalation conduit (16) defining an exhalation passage, the exhalation passage configured to allow passage of a gaseous or fluid medium exhaled from the patient, the gaseous or fluid medium comprising droplets of the liquid medicament; and
a single-piece filter (20) having an internal volume between 5 mL and 200 mL and an axial length between 7.5 cm and 30 cm connected to the exhalation conduit (16), the single-piece filter (20) configured to capture at least some of the droplets of the liquid medicament from the gaseous or fluid medium, wherein:
the single-piece filter (20) comprises a continuous, single-piece filter body (22) having a proximal portion (24) and a distal portion (26),
the proximal portion (24) of the filter body (22) has a proximal end (28) that defines an opening having a diameter between 15 mm and 30 mm configured to connect to the exhalation conduit (16), and
the distal portion (26) of the filter body has a closed distal end (30) that does not allow any gaseous or fluid medium to escape without passing through the single-piece aerosol exhalation filter (20).

2. The aerosol delivery device of claim 1, wherein the single-piece filter (20) has a generally tubular shape.

3. The aerosol delivery device of claim 1, wherein the opening defined by the proximal end (28) of the filter body (22) has a circular cross section.

4. The aerosol delivery device of claim 1, wherein the single-piece filter (20) is connected to the exhalation conduit (16) through a friction fit.

5. The aerosol delivery device of claim 1, wherein:
the inhalation passage is fluidly coupled to the nebulizer (12), and
the exhalation passage is fluidly coupled to the inhalation passage.

6. The aerosol delivery device of claim 1, wherein the single-piece filter (20) is made of a microporous material.

7. The aerosol delivery device of claim 6, wherein the microporous material is capable of filtering at least 95% of airborne particles with aerodynamic diameters of 0.3 micrometres or greater.

8. The aerosol delivery device of claim 1, further comprising an exhaust port located at one end of the exhalation conduit (16), wherein the opening of the proximal portion (24) of the filter body (22) is configured to connect to the exhaust port.

9. The aerosol delivery device of claim 1, wherein a shape of a cross section of the exhalation conduit (16) is the same as a shape of a cross-section of the opening of the proximal portion (24) of the filter body (22).

10. The aerosol delivery device of claim 1, wherein the gaseous or fluid medium is a patient's breath.

## Patentansprüche

1. Aerosol-Abgabevorrichtung zur Abgabe eines Aerosols an einen Patienten, wobei die Aerosol-Abgabevorrichtung umfasst:
einen Vernebler (12), der ausgelegt ist, ein Aerosol zu erzeugen, das Tröpfchen eines flüssigen Medikaments enthält;
eine Inhalationsleitung (18), die einen Inhalationsdurchgang definiert, wobei der Inhalationsdurchgang ausgelegt ist, den Durchgang des von dem Vernebler (12) erzeugten Aerosols zu dem Patienten zu gestatten;
eine Exhalationsleitung (16), die einen Exhalationsdurchgang definiert, wobei der Exhalationsdurchgang ausgelegt ist, den Durchgang eines gasförmigen oder
fluiden Mediums zu gestatten, das von dem Patienten ausgeatmet wird, wobei das gasförmige oder fluide Medium Tröpfchen des flüssigen Medikaments umfasst; und
einen einteiligen Filter (20) mit einem internen Volumen zwischen 5 ml und 200 ml, und einer axialen Länge zwischen 7,5 cm und 30 cm, der mit der Exhalationsleitung (16) verbunden ist, wobei der einteilige Filter (20) ausgelegt ist, mindestens einige der Tröpfchen des flüssigen Medikaments aus dem gasförmigen oder fluiden Medium abzufangen, wobei:
der einteilige Filter (20) einen kontinuierlichen, einteiligen Filterkörper (22) mit einem proximalen Abschnitt (24) und einem distalen Abschnitt (26) umfasst,
der proximale Abschnitt (24) des Filterkörpers (22) ein proximales Ende (28) aufweist, das eine Öffnung mit einem Durchmesser zwischen 15 mm und 30 mm definiert, die ausgelegt ist, an die Exhalationsleitung (16) angeschlossen zu werden, und
der distale Abschnitt (26) des Filterkörpers ein geschlossenes distales Ende (30) aufweist, das nicht gestattet, dass irgendein gasförmiges oder fluides Medium entweicht, ohne durch den einteiligen Aerosol-Exhalationsfilter hindurchzugehen.

2. Aerosol-Abgabevorrichtung nach Anspruch 1, wobei der einteilige Filter (20) eine allgemein rohrförmige Gestalt aufweist.

3. Aerosol-Abgabevorrichtung nach Anspruch 1, wobei die Öffnung, die von dem proximalen Ende (28) des Filterkörpers (22) definiert wird, einen kreisförmigen Querschnitt aufweist.

4. Aerosol-Abgabevorrichtung nach Anspruch 1, wobei der einteilige Filter (20) mit der Exhalationsleitung (16) durch eine Reibungspassung verbunden ist.

5. Aerosol-Abgabevorrichtung nach Anspruch 1, wobei:
der Inhalationsdurchgang mit dem Vernebler (12) fluidisch gekoppelt ist, und
der Exhalationsdurchgang mit dem Inhalationsdurchgang fluidisch gekoppelt ist.

6. Aerosol-Abgabevorrichtung nach Anspruch 1, wobei der einteilige Filter (20) aus einem mikroporösen Material hergestellt ist.

7. Aerosol-Abgabevorrichtung nach Anspruch 6, wobei das mikroporöse Material mindestens 95 % Schwebeteilchen mit aerodynamischen Durchmessern von 0,3 Mikrometern oder mehr filtern kann.

8. Aerosol-Abgabevorrichtung nach Anspruch 1, ferner umfassend einen Auslassport, der an einem Ende der Exhalationsleitung (16) angeordnet ist, wobei die Öffnung des proximalen Abschnitts (24) des Filterkörpers (22) ausgelegt ist, an den Auslassport angeschlossen zu werden.

9. Aerosol-Abgabevorrichtung nach Anspruch 1, wobei eine Form eines Querschnitts der Exhalationsleitung (16) gleich ist wie eine Form eines Querschnitts der Öffnung des proximalen Abschnitts (24) des Filterkörpers (22).

10. Aerosol-Abgabevorrichtung nach Anspruch 1, wobei das gasförmige oder fluide Medium der Atem eines Patienten ist.

## Revendications

1. Dispositif de délivrance d'un aérosol pour délivrer un aérosol à un patient, le dispositif de délivrance d'un aérosol comprenant :
un nébuliseur (12) configuré pour générer un aérosol contenant des gouttelettes d'un médicament liquide ;
un conduit d'inhalation (18) définissant un passage d'inhalation, le passage d'inhalation étant configuré pour permettre un passage de l'aérosol généré par le nébuliseur (12) au patient ;
un conduit d'expiration (16) définissant un passage d'expiration, le passage d'expiration étant configuré pour permettre un passage d'un milieu gazeux ou liquide expiré par le patient, le milieu gazeux ou liquide comprenant des gouttelettes du médicament liquide ; et
un filtre monobloc (20) présentant un volume interne compris entre 5 mL et 200 mL et une longueur axiale comprise entre 7,5 cm et 30 cm relié au conduit d'expiration (16), le filtre monobloc (20) étant configuré pour capturer au moins certaines des gouttelettes du médicament liquide à partir du milieu gazeux ou liquide, dans lequel :
le filtre monobloc (20) comprend un corps de filtre monobloc (22), continu, présentant une partie proximale (24) et une partie distale (26),
la partie proximale (24) du corps de filtre (22) présente une extrémité proximale (28) qui définit une ouverture présentant un diamètre compris entre 15 mm et 30 mm configuré pour se connecter au conduit d'expiration (16), et
la partie distale (26) du corps de filtre présente une extrémité distale (30) obturée qui ne permet pas à un milieu gazeux ou liquide de s'échapper sans passer à travers le filtre monobloc (20) d'expiration d'aérosol.

2. Dispositif de délivrance d'un aérosol selon la revendication 1, dans lequel le filtre monobloc (20) présente une forme générale tubulaire.

3. Dispositif de délivrance d'un aérosol selon la revendication 1, dans lequel l'ouverture définie par l'extrémité proximale (28) du corps de filtre (22) présente une section transversale circulaire.

4. Dispositif de délivrance d'un aérosol selon la revendication 1, dans lequel le filtre monobloc (20) est relié au conduit d'expiration (16) par un ajustement par friction.

5. Dispositif de délivrance d'un aérosol selon la revendication 1, dans lequel :
le passage d'inhalation est en couplage de fluide avec le nébuliseur (12), et
le passage d'expiration est en couplage de fluide avec le passage d'inhalation.

6. Dispositif de délivrance d'un aérosol selon la revendication 1, dans lequel le filtre monobloc (20) est fabriqué en un matériau microporeux.

7. Dispositif de délivrance d'un aérosol selon la revendication 6, dans lequel le matériau microporeux est apte à filtrer au moins 95 % des particules atmosphériques présentant des diamètres aérodynamiques de 0,3 micromètres ou plus.

8. Dispositif de délivrance d'un aérosol selon la revendication 1, comprenant en outre un raccordement d'échappement situé à une extrémité du conduit d'expiration (16), dans lequel l'ouverture de la partie proximale (24) du corps de filtre (22) est configurée pour se connecter au raccordement d'échappement.

9. Dispositif de délivrance d'un aérosol selon la revendication 1, dans lequel une forme d'une section transversale du conduit d'expiration (16) est identique à une forme d'une section transversale de l'ouverture de la partie proximale (24) du corps de filtre (22).

10. Dispositif de délivrance d'un aérosol selon la revendication 1, dans lequel le milieu gazeux ou liquide est une respiration d'un patient.
